# EUROPEAN PATENT SPECIFICATION

(11) **EP 3 400 938 B1**
(45) Date of publication and mention of the grant of the patent: **09.09.2020**
(21) Application number: 18169956.2
(22) Date of filing: 27.04.2018
(51) Int. Cl.: A61K 31/4192, A61P 35/00

(54) **NOVEL N-(1,2,3-TRIAZOLMETHYL)ISATIN AND N-(1,2,3-TRIAZOLMETHYL)-3-HYDROXY-3-ARYLOXINDOLES WITH CYTOTOXIC AND ANTI-TUMOR ACTIVITY**
NEUARTIGE N-(1,2,3-TRIAZOLMETHYL)ISATIN- UND N-(1,2,3-TRIAZOLMETHYL)-3-HYDROXY-3-ARYLOXINDOLE MIT ZYTOTOXISCHER UND ANTITUMORALER AKTIVITÄT
NOUVEAUX N-(1,2,3-TRIAZOLMÉTHYL) ISATINE ET N-(1,2,3-TRIAZOLMÉTHYL)-3-HYDROXY-3-ARYLOXINDOLES AYANT UNE ACTIVITÉ CYTOTOXIQUE ET ANTITUMORALE

(30) Priority: 08.05.2017 PT 11006317
(43) Date of publication of application: 14.11.2018
(73) Proprietor: Universidade de Évora, 7000-803 Évora (PT)
(72) Inventor: BURKE, Anthony, 7005-288 Évora (PT); GAUDIO, Eugenio, CH-6500 Bellinzona (CH); BUSTO VAZQUEZ, Natalia, 09002 Burgos (ES); BERTONI, Francesco, CH-6500 Bellinzona (CH); SILVA MARQUES, Carolina, 2475-150 Benedita (PT)
(74) Representative: Ferreira, Maria Silvina

(56) References cited:
- CAROLINA S. MARQUES ET AL: "Enantioselective Rhodium(I)-Catalyzed Additions of Arylboronic Acids to N -1,2,3-Triazole-Isatin Derivatives: Accessing N -(1,2,3-Triazolmethyl)-3-hydroxy-3-aryloxi ndoles", CHEMCATCHEM, vol. 8, no. 22, 4 October 2016 (2016-10-04), pages 3518-3526, XP055491169, DE ISSN: 1867-3880, DOI: 10.1002/cctc.201600901
- SINGH ET AL.: "AZIDE-ALKYNE CYCLOADDITION EN ROUTE TO NOVEL 1H-1,2,3-TRIAZOLE TETHERED ISATIN CONJUGATES WITH IN VITRO CYTOTOXIC EVALUATION", EUROPEAN JOURNAL OF MEDICINAL CHEMISTRY, vol. 55, 2012, pages 455-461, XP002784801,

## Description

### Technical domain

The invention relates to the use of novel chiral *N-*(1,2,3-triazolmethyl)-3-hydroxy-3-aryloxindoles and *N*-(1,2,3-triazolmethyl)-isatin derivatives with cytotoxic and anti-tumor activity.

### Background

The present invention relates to the use of novel chiral *N-*(1,2,3-triazolmethyl)-3-hydroxy-3-aryloxindoles and *N-*(1,2,3-triazolmethyl)-isatin derivatives with cytotoxic and anti-tumor activity.
At the present time, most drugs either on the market or in the pipeline are chiral, and generally exist in one enantiomeric form, as in most cases only one of the enantiomers is effective, in some cases the other enantiomer nullifies the effect of the active enantiomer, or it may have very harmful effects on the organism (NG, 2004). 3-Substituted 3-hydroxyoxindoles exhibit broad ranging biological activities, that include SSR-149415 an orally active non-peptide vasopressin receptor antagonist (Gal *et al*. **2002**), AG-041R a gastrin/CCK-B receptor antagonist and effective in repairing cartilage defects (Ochi *et al*. 2001), an *N*-methyl-spiro[2.3']oxindolespiro[3.2"]-5,6-dimethoxy-1"-indanone-4-(arylsubstituted) pyrrolidine which was identified as an acetylcholinesterase (AChE) inhibitor (Ali *et al*. 2010) and NITD609 an antimalarial drug candidate 1

(Rottmann et al. 2010). We have developed a family of 3-substituted-2-hydroxy oxindole compounds which manifest good butyrylcholinesterase inhibition (Totobenazara *et al*. 2016). On the other hand many biologically active compounds contain a 1,2,3-triazole moiety (Burke and Marques 2015, Thirumurugan *et al.* 2013, Tron *et al.* 2007 and Totobenazara and Burke 2015). For example; carboxyamido-triazole (CAI), a signal transduction inhibitor used in cancer treatment (Perabo *et al*. 2004), the β-lactam antibiotic tazobactum (Yang *et al*., 1999) and the cephalosporin celfatrizine (Neu and Fu, 1979).
The synthesis of these molecules has previously been discussed in the provisional patent application 109598 (Burke and Marques 2016).

Document "Enantioselective Rhodium (I)-Catalyzed additions of Arylboronic Acids to N-1,2,3-Triazole-Isatin Derivatives: Acessing N-(1,2,3-Triazolmethyl)-3-hydroxy-3-aryloxindoles", Chemcatchem, vol. 8, no. 22, 2016, discloses a catalytic asymmetric route to produce unknown families of *N*-(1,2,3-Triazolmethyl)-3-hydroxy-3-phenyloxindoles using rhodium catalysts. In this document, the anti-tumor activity of such compounds is already predicted. However, the document does not present activity data or mentions which types of tumor cells can be inhibited with the compounds.

Thus, the present invention provides new families of *N-*(1,2,3-triazolmethyl)-3-hydroxy-3-aryloxindoles and *N-*(1,2,3-triazolmethyl)-isatin derivatives with anti-cancer properties.

### Summary

It is the goal of this application to report the use of new families of *N*-(1,2,3-triazolmethyl)-3-hydroxy-3-aryloxindoles and *N*-(1,2,3-triazolmethyl)-isatin derivatives which have potent activity against colon adenocarcinoma and lymphoma cells.
Thus, this application describes compounds of formula (**I**) 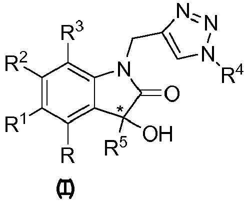 in which, R, R¹, R² and R³ represent; H, alkyl, aryl, vinyl, allyl, alkoxyl, halogen (F, Br, Cl), OH, CN, CHO and CO₂H, R⁴ represents a linear alkyl group with 1-6 carbons, a cycloheteroalkyl group, like: 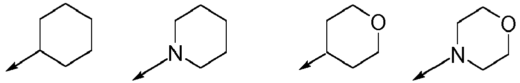 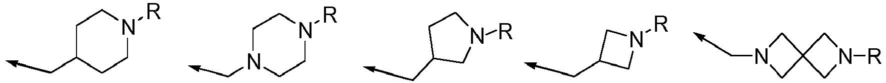 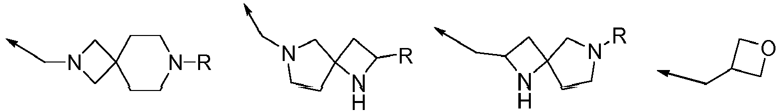 an aryl group or heteroaryl group, that includes, a substituted phenyl group (containing alkoxyl, halogen (F, Br, Cl), OH, CN, CHO, CO₂H), 2- and 4-pyridine, pyrimidine, pyridazine, thiophene, furane, pyran, benzoyl, pyrrole. A benzyl group 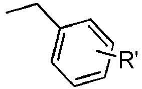
Where R' is alkoxyl, halogen (F, Br, Cl), OH, CN, CHO and CO₂H,
R⁵ represents an aryl group.

All these compounds can be accessed in one enantiomeric form - either pure or enriched.

The compounds of formula (**I**) are obtained from compounds of formula (**II**).

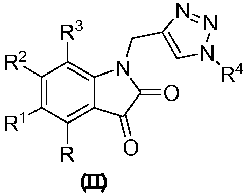

Introduction of the 1,2,3-triazole unit in (**II**) is easily accomplished using the highly efficient Sharpless-Meldal Click reaction.
Considering that these compounds contain two potent pharmacophores, namely a 3-aryl-3-hydroxyoxindole and a 1,2,3-triazole unit, they have been shown to exhibit potent cytotoxic activity against SW480 tumor cells.

### General description

It is the goal of this application to report the use of chiral *N*-(1,2,3-triazolmethyl)-3-hydroxy-3-aryloxindoles and *N*-(1,2,3-triazolmethyl)-isatin derivatives with cytotoxic and anti-tumor activity.
The compounds of formula (**I**) are obtained from isatin compounds of formula (**II**) by metal catalysed addition of arylboronic acids, esters or trifluoroborate reagents (Burke and Marques 2016).

The substrates of formula (**II**) are obtained from N-propargyl isatin precursors and then subjected to the Sharpless-Meldal dipolar cycloadditon click-reaction with appropriate organic azides (Rostovtsev *et al.* 2002, Tornøe *et al.* 2002). These reactions are conducted under standard copper catalysed conditions (Burke and Marques 2016) (Scheme 1).

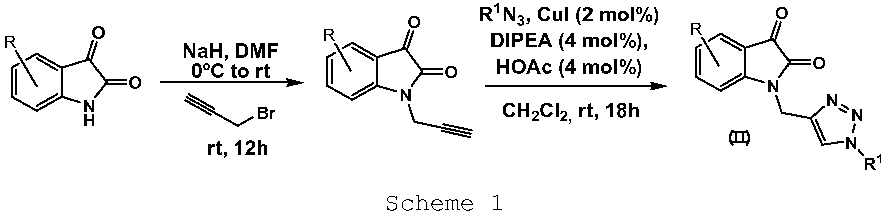

The asymmetric catalytic arylation of (**II**) to (**I**) is achieved using an appropriate chiral transition metal containing catalyst that contains either Rh (preferable), Pd, Ru, Ir or Cu. The reactions are normally conducted by simply adding the pre-catalyst and the chiral ligand to the substrate (like) in a flask under an inert gas atmosphere in the presence of a borane reagent and a base (Scheme 1).
In the case of the Rh catalyzed reactions generally the following commercially available pre-catalysts are used: [Rh(COD)Cl]₂, [Rh(COD)OH]₂, [Rh(nbd)Cl]₂, Rh(COD)₂BF₄, [Rh(C₂H₄)₂Cl]₂, Rh(acac)(coe)₂ and Rh(acac)(eth)₂.
The quantity of pre-catalyst varies between 1 and 10 mol%. The chiral ligands that can be used include: (*R*)- and (*S*)-BINAP, TolBINAP, DIOP, DIPAMP, DioxPhos, DeguPhos, Xylyl-P-Phos, (*R*)-Phanephos, (*R*)*-*ShiP*,* (*R*)-SIPHOS, (*R, R*)-Chiraphos, C₃-TunePhos, TangPhos, BINAPINE, BINAPHANE, PhanePhos, Synphos, DuanPhos, Walphos, Josiphos, Naud, SchmalzPhos, QuinNap, phosphoramidite derivatives (MonoPhos™) etc, chiral NHCs, dienes like; *C₂*-Symmetric Bicyclo[2.2.2]octadiene and derivatives, chiral sulfinamide-alkene ligands and oxazoline based ligands, like: PyBox, Pyrox, Quinox.
The quantity of chiral ligand varies between 1 and 10 mol%.

In the case of the base, the following are generally used: triethylamine, K₂CO₃, Na₂CO₃, CaCO₃, Ba(OH)₂, KOAc, DIPEA, K₃PO₄, NMM, DBU, KOH, KF, Cs₂CO₃ and KOtBu.
The quantity of base used ranges from 0.1 to 2 equivalents. Various commercial arylboronic acids, arylboronic esters and trifluoroborate salts can be used.
The borane loading ranges from 1 to 3 equivalents.
The following reaction solvents can be used: toluene, dimethyl ether, diethyl ether, dichloroethane, THF, 1,4-dioxane, acetone, acetonitrile, methanol, ethanol, isopropanol, 1,2-dichloroethane, DMF, DMA and NMP.

The reactions were run under an inert atmosphere (e.g. under dry nitrogen or argon).
The reactions are generally run at temperatures of between 25 °C and 100 °C. High conversions of up to 99% and enantioselectivities of 97% ee can be achieved. The reaction times varied from between 10 and 24 hours.

On the basis of previous studies with an analogous compound (Totobenazara *et al*. 2016) in the case of the reactions using BINAP the (S)-configuration was tentatively assigned to the newly created stereocenter when (*R*)-BINAP was used and the (*R*)-configuration when (*S*)-BINAP was used.

Some examples are shown in Table 1 and Figure 1.

**Table 1. Rhodium-catalysed asymmetric addition of phenylboronic acid to N-triazole-benzyl isatin derivative to give (1).**

| | | | | |
|---|---|---|---|---|
| 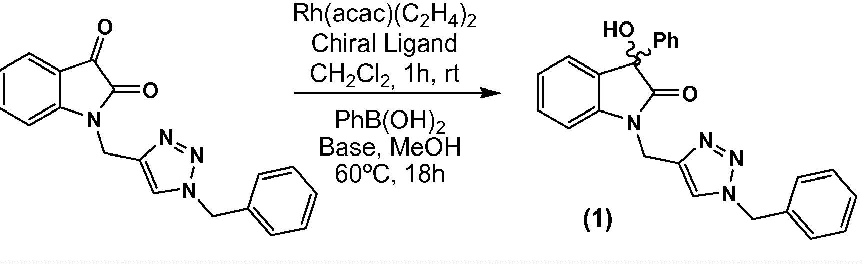 | | | | |

| **Entry^{[a]}** | **Chiral Ligand** | **Base** | **Conversion^{[b]} /%** | **ee^{[c]}/%** |
|---|---|---|---|---|
| 1 | (*R*)-BINAP | DIPEA | 89 | 87 (*S*) |
| 2 | (*R*)-BINAP | NEt₃ | <10 | 94 (*S*) |
| 3 | (*R*)-BINAP | KO*t*Bu | 16 | 97 (*S*) |
| 4 | (*S*)-BINAP | DIPEA | 31^{[g]} | 74 *(R)* |
| 5 | (*R*)-Tolyl-BINAP | DIPEA | <10 | 21 |
| 6 | (*R*)-MonoPhos | DIPEA | 77 | *rac* |
| 7 | NHC Chiral | DIPEA | 46 | *rac* |
| 8 | (*S,S*)-Diene | DIPEA | <5 | 22 |
| 9 | (*R*)-PhanePhos | DIPEA | <10 | 13 |
| 10^{[d]} | (*R*)-BINAP | NEt₃ | <10 | 56 (*S*) |
| [a] Reaction conditions: 3 mol% Rh(acac) (C₂H₄)₂, 6 mol% Chiral Ligand (see ligand structure in Scheme 4), CH₂Cl₂, 1h, rt; 0.16 mmol **(2a)**, 0.32 mmol PhB(OH)₂, 0.08 mmol Base, MeOH (2 mL), 60°C, 18h. [b] and [c] Determined by HLPC with chiral stationary phase. [d] Toluene was used as solvent. [e] Acetone was used as solvent. [f] Reaction run at room temperature. [g] Isolated yield. | | | | |

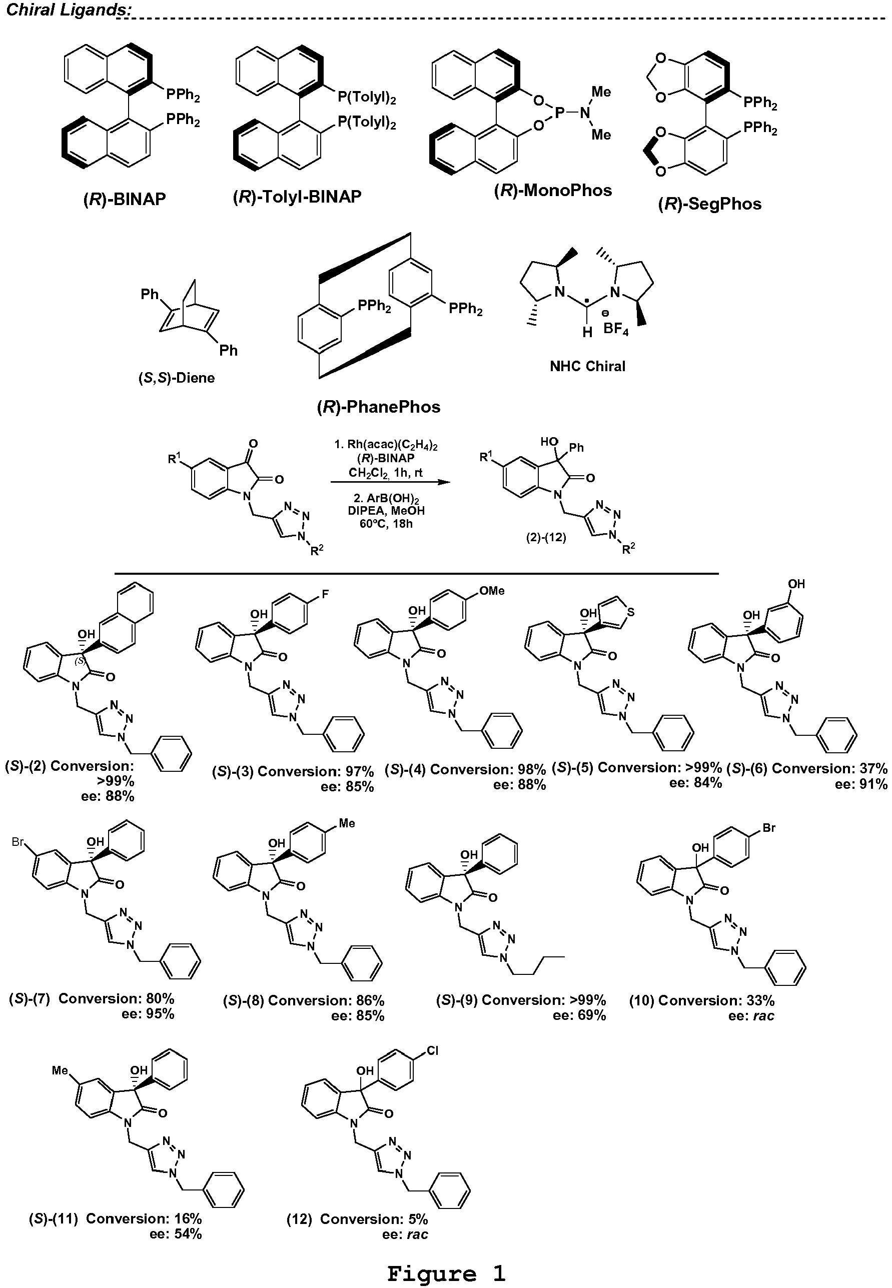

One of the main strategies for the treatment of cancer is chemotherapy (De Santis *et al*. 2014). There are two main factors that made the development of new chemotherapeutic agents crucial: the side effects of the current anticancer drugs used in the clinic and the appearance of tumour cell resistance.
A first step facing at tackling this challenging goal is the evaluation of the cytotoxic activity of the new synthesized drugs. In our case, cytotoxicity was evaluated by means of the antiproliferative assay MTT, which is a high-throughput method widely used for the screening of new drugs that is based on the ability of the mitochondrial dehydrogenases to reduce a water soluble yellow dye, namely 3-(4,5-dimethylthiazol-2-yl)-2,5-diphenyl tetrazolium bromide, to form a water insoluble blue formazan (McCauley *et al.*, **2013**). Cytotoxic studies using the MTT assay with colon adenocarcinoma cells (SW480 cell line) showed that a number of these compounds showed better anti-proliferative activity that the bench-mark doxorubicin (Dox) (Table 2 entry 17) which is a known DNA intercalator and which is commonly used to treat some leukemias and Hodgkin's lymphoma, including cancers of the bladder, breast, stomach, lung, ovaries, thyroid, soft tissue sarcoma, multiple myeloma, and others (Rossi, 2013) (Table 2). However, doxorubicin shows severe systemic toxicity, which has limited its use in the clinical treatment of cancer (Xu *et al*. 2003), and thus there is a need for alternative drugs to be used on their own or in combination with doxorubicin.
The best inhibition was obtained with (*S*)-**2** (Table 2, entry 2). Overall the (S) series gave better inhibition results.

**Table 2 IC₅₀ values obtained from MTT assay with a 24h exposure time**

| **Entry** | **Compound** | **Enantiopurity (ee%)** | **IC₅₀ µM** |
|---|---|---|---|
| 1 | ISATIN-MENTHOL | | 13.7 |
| 2 | ISATIN-BENZYL | | 84.4 |
| 3 | (*S*) **-1** | 80 | 63 |
| 4 | (*S*) -**2** | 88 | 12.8 |
| 5 | (*S*) -**3** | 78 | 25.9 |
| 6 | (*S*) -**4** | 87 | 48.9 |
| 7 | (*S*) -**5** | 75 | 33.0 |
| 8 | (*S*) -**6** | 76 | 52.8 |
| 9 | (*S*) -**7** | 91 | 34.1 |
| 10 | (*S*) -**8** | 85 | >200 |
| 11 | (*S*) -**9** | 82 | >200 |
| 12 | (*R*) *-***1** | 74 | 82.6 |
| 13 | (*R*) -**2** | 86 | 18.6 |
| 14 | (*R*) *-***3** | 76 | 49.1 |
| 15 | (*R*) -**4** | 87 | 76.4 |
| 16 | (*R*) *-***5** | 89 | 86.4 |
| 17 | (*R*) -**6** | 94 | 210.2 |
| 18 | (*R*) -**7** | 92 | 42.5 |
| 19 | Dox | | 65.30 (Xu *et al*. 2003) |

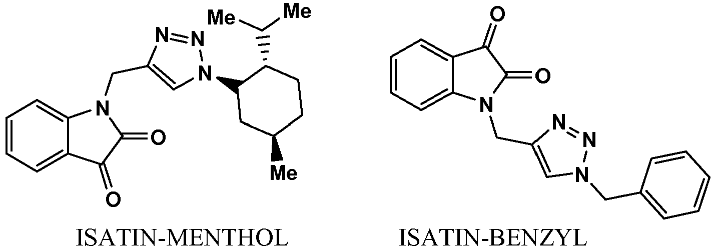

A set of ten compounds was tested against four lymphoma cell lines belonging to Germinal Center B-cell-like Diffuse Large B-Cell lymphoma, GCB-DLBCL and Activated B-Cell-like Diffuse Large B-Cell lymphoma, ABC-DLBCL (Table 3). Compounds were tested in triplicates as single agents, for 72 hours by using a large range of concentrations. Among all compounds, ISATIN-MENTHOL and ISATIN-BENZYL showed the best anti-lymphoma activity against two ABC-DLBCL cell lines (OCI-LY-10) and (SU-DHL-2). In particular, ISATIN-BENZYL is the most promising one reaching IC₅₀s of 1 and 5 µM in the OCI-LY-10 and SU-DHL-2 cell lines (Table 3).

**Table 3. IC₅₀ values obtained from MTT assay with a 72h exposure time on lymphoma cell lines.**

| | | **GCB-DLBC cell lines IC₅₀, µM** | | **ABC-DLBC cell lines IC₅₀, µM** | |
|---|---|---|---|---|---|
| **Entry** | **Compound** | DOHH-2 | VAL | OCI-LY-10 | SU-DHL-2 |
| 1 | ISATIN-MENTHOL | >20 | >20 | 7 | 15 |
| 2 | ISATIN-BENZYL | >20 | >20 | 1 | 5 |
| 3 | (*R*) *-***1** | >20 | >20 | >20 | >20 |
| 4 | (*R*) *-***2** | >20 | >20 | >20 | >20 |
| 5 | (*S*) -**2** | >20 | >20 | >20 | 19 |
| 6 | (*R*) *-***3** | >20 | >20 | >20 | >20 |
| 7 | (*S*) -**3** | >20 | >20 | >20 | >20 |
| 8 | (*R*) *-***5** | >20 | >20 | >20 | >20 |
| 9 | (*S*) -**7** | 19 | >20 | >20 | 19 |

### Examples

### Synthesis of the N-Propargylisatin substrates

### General Procedure

To a solution of isatin or its derivative in DMF was added NaH (1.5 equiv.) at 0 °C and the mixture was stirred for about 10 min. A purple solution can be seen in the reaction flask. The mixture was left stirring at room temperature for one additional hour, and then the propargyl bromide (1.1 equiv.) was added dropwise. The mixture was stirred for 12h at room temperature. The DMF was removed under reduced pressure and AcOEt and H₂O were added. The aqueous layer was extracted with AcOEt (3×30 mL) and the combined organic layers were washed with *brine,* dried over MgSO₄ and evaporated to give the desired *N*-propargyl products.

### 1-(Prop-2-ynyl)indoline-2,3-dione

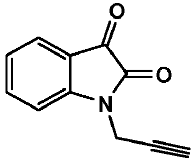

Orange solid (50% yield). **¹H NMR (CDCl₃, 400 MHz) δ:** 2.32-2.33 (m, 1H, CH), 4.55 (s, 2H, CH₂), 7.14-7.21 (m, 2H, Ar), 7.64-7.68 (m, 2H, Ar). **¹³C NMR (CDCl₃, 100 MHz) δ:** 29.56, 73.46, 75.80, 111.21, 117.79, 124.32, 125.57, 138.56, 149.72, 157.27, 182.65.

### 5-Methyl-1-(prop-2-ynyl)indoline-2,3-dione

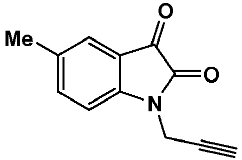

Orange solid (21% yield) . **¹H NMR (CDCl₃, 400 MHz) δ:** 2.29 (s, 1H, CH), 2.35 (s, 3H, CH₃), 4.51 (s, 2H, CH₂), 7.01-7.03 (d, *J*= 8 Hz, 1H, Ar), 7.43-7.45 (m, 2H, Ar). **¹³C NMR (CDCl₃, 100 MHz) δ:** 20.86, 29.56, 73.33, 75.93, 111.02, 117.83, 125.93, 134.23, 138.98, 147.57, 157.45, 182.94.

### 5-Bromo-1-(prop-2-ynyl)indoline-2,3-dione

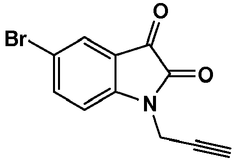

Orange solid (30% yield) . **¹H NMR (CDCl₃, 400 MHz) δ:** 2.33 (s, 1H, CH), 4.53 (s, 2H, CH₂), 7.04-7.06 (d, *J*= 8 Hz, 1H, Ar), 7.29-7.35 (m, 1H, Ar), 7.75 (s, 1H, Ar). **¹³C NMR (CDCl₃, 100 MHz) δ:** 29.71, 73.91, 75.35, 112.98, 117.29, 118.95, 128.37, 140.78, 148.40, 156.53, 181.51.

### Synthesis of the N-(1,2,3-Triazolmethyl)-isatin substrates General Procedure

In a round-bottomed flask were added *N*-propargyl isatin derivative, the azide (1 equiv.), CuI (0.02 equiv.), DIPEA (0.04 equiv.), HOAc (0.04 equiv.) and CH₂Cl₂. The mixture was stirred at room temperature for 18 h, and monitored by TLC. The reaction mixture was filtered with a sinitered glass funnel with a *celite* layer and washed with CH₂Cl₂. The solvent was evaporated on a rotary evaporator and the crude product mixture was purified by silica gel chromatography using a gradient of Hexane/AcOEt (5/1)-(1/1)- AcOEt.

### 1-((1-Benzyl-1H-1,2,3-triazol-4-yl)methyl)indoline-2,3-dione (ISATIN-MENTHOL)

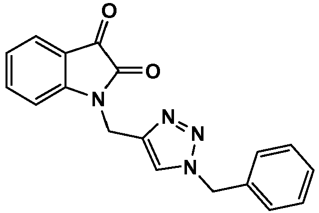

Orange solid (80% yield). **m.p.=** 137.2-138.8°C. **¹H NMR ((CD₃)₂CO, 400 MHz) δ:** 5.02 (s, 2H, CH₂), 5.60 (s, 2H, CH₂), 7.13-7.17 (t, *J*= 8 Hz, 1H, Ar), 7.22-7.24 (d, *J*= 8 Hz, 1H, Ar), 7.32-7.36 (m, 5H, Ar), 7.54-7.56 (d, *J*= 8 Hz, 1H, Ar), 7.62-7.66 (t, *J*= 8 Hz, 1H, Ar), 8.04 (s, 1H, =CH). **¹³C NMR ((CD₃)₂CO, 100 MHz) δ:** 36.06, 54.26, 112.21, 118.70, 124.04, 124.29, 125.14, 128.87, 129.11, 129.66, 136.86, 138.93, 143.06, 151.67, 158.53, 184.13.

### 1-((1-Butyl-1H-1,2,3-triazol-4-yl)methyl)indoline-2,3-dione

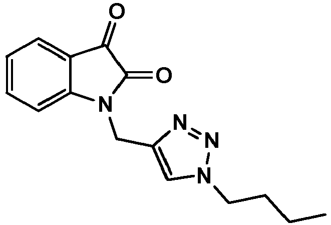

Orange solid (20% yield). **m.p. =** 104.2-105.7°C. **¹H NMR ((CD₃)₂CO, 400 MHz) δ:** 0.86-0.90 (t, *J*= 8Hz, 3H, CH₃), 1.25-1.31 (q, *J*= 8 Hz, 2H, CH₂), 1.79-1.86 (m, 2H, CH₂), 4.35-4.39 (t, *J*= 8 Hz, 2H, CH₂), 5.02 (s, 2H, CH₂), 7.13-7.16 (t, 1H, Ar), 7.23-7.25 (d, *J*= 8 Hz, 1H, Ar), 7.53-7.55 (d, *J*= 8 Hz, 1H, Ar), 7.62-7.66 (t, *J*= 8 Hz, 1H, Ar), 8.02 (s, 1H, =CH). **¹³C NMR ((CD₃)₂CO, 100 MHz) δ:** 13.65 (CH₃), 20.18 (CH₂), 32.88 (CH₂), 36.04 (CH₂), 50.36 (CH₂), 112.21 (CH), 118.60 (C), 123.79 (CH), 124.28 (CH), 125.13 (CH), 138.94 (CH), 142.51 (C), 151.64 (C), 158.49 (C=O), 184.16 (C=O). **MS (ESI) m/z:** 285.0 [M]⁺.

### 1-((1-Cyclohexyl-1H-1,2,3-triazol-4-yl)methyl)indoline-2,3-dione

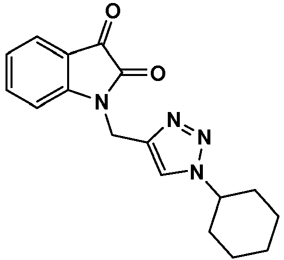

Orange oil (10% yield). **¹H NMR ((CD₃)₂SO, 400 MHz) δ:** 1.17-1.23 (m, 1H, CH₂), 1.34-1.37 (m, 2H, CH₂), 1.62-1.79 (m, 5H, CH₂), 1.98-2.01 (m, 2H, CH₂), 4.39-4.45 (m, 1H, CH), 4.94 (s, 2H, CH₂), 7.10-7.17 (m, 2H, Ar), 7.55-7.57 (d, *J*= 8 Hz, 1H, Ar), 7.61-7.65 (t, *J*= 8 Hz, 1H, Ar), 8.20 (s, 1H, =CH). **¹³C NMR ((CD₃)₂SO, 100 MHz) δ:** 24.54 (CH₂), 24.67 (CH₂), 32.83 (CH₂), 35.17 (CH₂), 59.05 (CH), 111.17 (CH), 117.62 (C), 121.52 (CH), 123.36 (CH), 124.45 (CH), 138.09 (CH), 141.16 (C), 150.25 (C), 157.80 (C=O), 183.15 (C=O). **MS (ESI) m/z:** 311.1 [M]⁺.

### 1-((1-((1R,2R,5S)-2-Isopropyl-5-methylcyclohexyl)-1H-1,2,3-triazol-4-yl)methyl)indoline-2,3-dione (ISATIN-BENZYL)

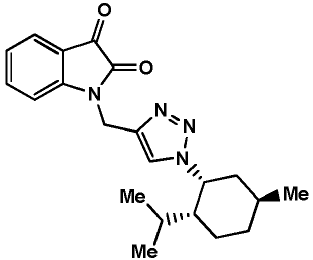

Orange solid (30% yield). **m.p.=** 155.2-156.7°C. **¹H NMR (CDCl₃, 400 MHz) δ:** 0.69-0.71 (d, *J*= 8 Hz, 3H, CH₃), 0.76-0.78 (d, *J*= 8 Hz, 3H, CH₃), 0.81-0.83 (d, *J*= 8 Hz, 3H, CH₃), 0.97-1.04 (m, 2H, CH₂), 1.31-1.45 (m, 2H, CH₂), 1.65-1.68 (m, 1H, CH), 1.78-1.90 (m, 4H, CH₂, CH), 4.97 (s, 1H, CH), 4.99 (s, 2H, CH₂), 7.07-7.11 (t, *J*= 8 Hz, 1H, Ar), 7.33-7.35 (d, *J*= 8 Hz, 1H, Ar), 7.55-7.58 (m, 2H, Ar), 7.68 (s, 1H, =CH). **¹³C NMR (CDCl₃, 100 MHz) δ:** 20.48 (CH₃), 21.14 (CH₃), 22.28 (CH₃), 24.82 (CH₂), 26.35 (CH), 29.10 (CH), 34.55 (CH₂), 35.49 (CH₂), 40.63 (CH₂), 46.70 (CH), 59.62 (CH), 111.82 (CH), 117.57 (C), 124.04 (CH), 125.33 (CH), 138.70 (CH), 140.60 (C), 150.48 (C), 158.05 (C=O), 183.34 (C=O). **MS (ESI) m/z:** 367.2 [M]⁺.

### 1-((1-Benzyl-1H-1,2,3-triazol-4-yl)methyl)-5-methylindoline-2,3-dione

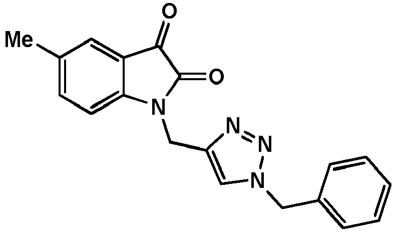

Orange solid (67% yield). **m.p.=** 143.1-144.0°C. **¹H NMR (CDCl₃, 400 MHz) δ:** 2.30 (s, 3H, CH₃), 4.96 (s, 2H, CH₂), 5.47 (s, 2H, CH₂), 7.18-7.20 (d, *J*= 8 Hz, 1H, Ar), 7.24-7.26 (m, 2H, Ar), 7.35-7.37 (m, 5H, Ar), 7.50 (s, 1H, =CH) . **¹³C NMR (CDCl₃, 100 MHz) δ:** 20.80 (CH₃) , 35.52 (CH₂), 54.54 (CH₂), 111.46 (CH), 117.64 (C), 122.90 (CH), 125.76 (CH), 128.40 (CH), 129.12 (CH), 129.35 (CH), 134.02 (C), 134.13 (C), 139.16 (CH), 142.34 (C), 148.17 (C), 158.17 (C=O), 183.47 (C=O). **MS (ESI) m/z:** 333.1 [M]⁺.

### 1-((1-Benzyl-1H-1,2,3-triazol-4-yl)methyl)-5-bromoindoline-2,3-dione

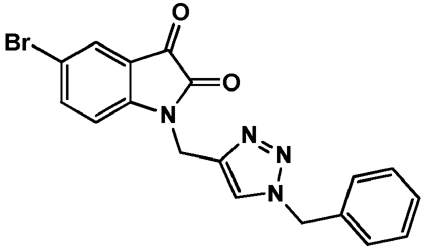

Orange solid (62% yield). **m.p.=** 173.1-175.0°C. **¹H NMR (CDCl₃, 400 MHz) δ:** 4.96 (s, 2H, CH₂), 5.47 (s, 2H, CH₂), 7.24-7.28 (m, 3H, Ar), 7.36 (m, 3H, Ar), 7.52 (s, 1H, =CH), 7.66-7.69 (m, 2H, Ar) . **¹³C NMR (CDCl₃, 100 MHz) δ:** 35.55 (CH₂), 54.57 (CH₂), 113.54 (CH), 117.07 (C), 118.75 (C), 122.97 (CH), 128.14 (CH), 128.43 (CH), 129.17 (CH), 129.37 (CH), 134.04 (C), 140.93 (CH), 141.80 (C), 149.04 (C), 157.27 (C=O), 182.08 (C=O). **MS (ESI) m/z:** 397.0 [M]⁺.

### Asymmetric Catalytic Synthesis of Chiral Non-Racemic N-(1,2,3-Triazolmethyl)-3-Hydroxy-3-Aryloxindoles

### General Procedure

In a round-bottomed flask under inert atmosphere were added Rh(acac) (C₂H₄)₂ (1.2 mg, 0.005 mmol, 3 mol%), (*R*)-BINAP (5.9 mg, 0.010 mmol, 6 mol%) and CH₂Cl₂ (1 mL). The mixture was stirred at room temperature for 1 hour, then the solvent was removed on a rotary evaporator. Under an inert atmosphere the following were added sequentially to the flask: *N-*triazole isatin substrate (0.16 mmol), the arylboronic acid (0.32 mmol, 2 equiv.), methanol (2 mL) and DIPEA (14.6 µL, 0.08 mmol, 0.5 equiv.). The mixture was stirred at 60°C during 18 h. The methanol was evaporated in rotary evaporator. To determine the isolated yields, the crude product was purified by silica gel chromatography using Hexane/AcOEt (1/1) as eluent to afford the desired product **(1)-(12).** To determine the conversions, the crude product was filtered in a porous plate glass filter with a layer of *celite* and a layer of silica gel and eluted with Et₂O. The solvent was evaporated and the crude mixture was analysed by HPLC.

### 1-((1-Benzyl-1H-1,2,3-triazol-4-yl)methyl)-3-hydroxy-3-phenylindolin-2-one (S)-(1)

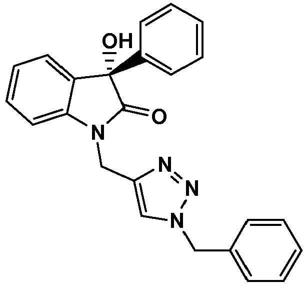

White solid. **m.p.=** 156.8-157.7 °C. **¹H NMR (CDCl₃, 400 MHz) δ:** 3.47 (s br, 1H, OH), 4.99 (s, 2H, CH₂), 5.37-5.49 (q, *J*= 16 Hz, 2H, CH₂), 7.04-7.07 (t, 1H, Ar), 7.19-7.34 (m, 13H, Ar), 7.42 (s, 1H, =CH). **¹³C NMR (CDCl₃, 100 MHz) δ:** 35.82, 54.38, 78.00, 110.18, 122.62, 123.86, 125.03, 125.41, 128.21, 128.45, 128.73, 128.96, 129.27, 130.14, 131.58, 134.39, 140.01, 142.18, 143.00, 177.34. **HRMS (ESI):** m/z calcd for C₂₄H₂₀N₄O₂ 396.15863, found for C₂₄H₂₁N₄O₂ [M] + 397.16590. **HPLC:** Daicel Chiralpak IA, n-hexane/i-propanol = 80/20, 1.0 mL/min, 210 nm, retention time: 22.707 min (minor), 33.933 min (major).

### 1-((1-Benzyl-1H-1,2,3-triazol-4-yl)methyl)-3-hydroxy-3-(naphthalen-2-yl)indolin-2-one (S)-(2)

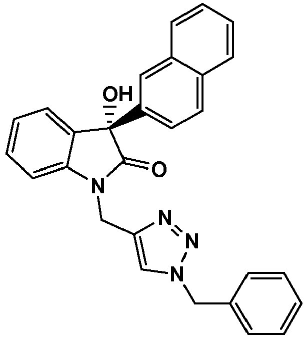

Pale orange solid. **m.p.=** 75.4-76.0 °C. **¹H NMR (CDCl₃, 400 MHz) δ:** 5.03 (s, 2H, CH₂), 5.30 (s br, 1H, OH), 5.37-5.48 (q, *J*= 16 Hz, 2H, CH₂), 7.05.7.08 (t, 1H, Ar), 7.19-7.35 (m, 9H, Ar), 7.44-7.48 (m, 3H, Ar), 7.69-7.71 (d, *J*= 8 Hz, 1H, Ar), 7.75-7.78 (m, 2H, Ar), 7.88 (s, 1H, =CH). **¹³C NMR (CDCl₃, 100 MHz) δ:** 35.89, 54.40, 78.21, 110.28, 122.65, 123.15, 123.93, 124.48, 125.12, 126.49, 126.54, 127.71, 128.22, 128.42, 128.73, 128.97, 129.28, 130.27, 131.50, 133.16, 133.21, 134.38, 137.30, 142.26, 143.02, 177.28. **MS (ESI) m/z:** 447.2 [M]⁺. **HPLC:** Daicel Chiralpak IA, n-hexane/i-propanol = 80/20, 1.0 mL/min, 210 nm, retention time: 35.513 min (minor), 39.607 min (major).

### 1-((1-Benzyl-1H-1,2,3-triazol-4-yl)methyl)-3-(4-fluorophenyl)-3-hydroxyindolin-2-one (S)-(3)

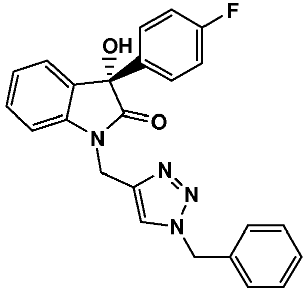

Pale orange oil. **¹H NMR ((CD₃)₂CO, 400 MHz) δ:** 4.91-5.07 (q, *J*= 16 Hz, 2H, CH₂), 5.58 (s, 2H, CH₂), 5.89 (s br, 1H, OH), 7.01-7.06 (m, 3H, Ar), 7.17-7.22 (m, 2H, Ar), 7.28-7.35 (m, 6H, Ar), 7.41-7.44 (m, 2H, Ar), 7.92 (s, 1H, =CH). **¹³C NMR ((CD₃)₂CO, 100 MHz) δ:** 35.89, 54.15, 77.85, 110.50, 115.48, 115.70, 123.75, 123.77, 125.36, 128.53, 128.62, 128.76, 129.04, 129.60, 130.26, 133.41, 136.78, 138.29, 138.32, 143.40, 143.57, 161.86, 164.29, 177.05. **MS (ESI) m/z:** 415.1 [M]⁺. **HPLC:** Daicel Chiralpak IA, n-hexane/i-propanol = 80/20, 1.0 mL/min, 210 nm, retention time: 19.673 min (minor), 23.127 min (major).

### 1-((1-Benzyl-1H-1,2,3-triazol-4-yl)methyl)-3-hydroxy-3-(4-methoxyphenyl)indolin-2-one (S)-(4)

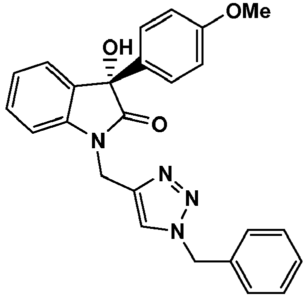

Pale orange solid. **m.p.=** 130.4-131.8 °C. **¹H NMR ((CD₃)₂SO, 400 MHz) δ:** 3.71 (s, 3H, OMe), 4.87-5.02 (q, *J*= 16 Hz, 2H, CH₂), 5.57 (s, 2H, CH₂), 6.68 (s, 1H, OH), 6.82-6.84 (d, *J*= 8 Hz, 2H, Ar), 7.01-7.04 (t, 1H, Ar), 7.13-7.19 (m, 4H, Ar), 7.26-7.37 (m, 6H, Ar), 8.10 (s, 1H, =CH). **¹³C NMR ((CD₃)₂SO, 100 MHz) δ:** 34.81, 52.79, 55.11, 76.60, 109.46, 113.50, 122.77, 123.52, 124.45, 126.74, 127.87, 128.15, 128.76, 129.10, 133.13, 133.26, 136.03, 142.06, 142.31, 158.73, 176.53. **MS (ESI) m/z:** 427.1 [M]⁺. **HPLC:** Daicel Chiralpak IA, n-hexane/i-propanol = 80/20, 1.0 mL/min, 210 nm, retention time: 32.767 min (minor), 38.927 min (major).

### 1-((1-Benzyl-1H-1,2,3-triazol-4-yl)methyl)-3-hydroxy-3-(thiophen-3-yl)indolin-2-one (S)-(5)

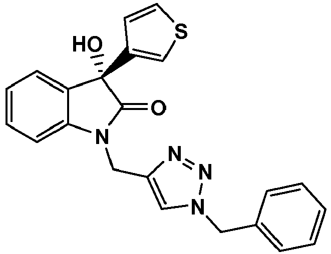

White solid. **m.p.=** 147.8-148.3 °C. **¹H NMR (CDCl₃, 400 MHz) δ:** 4.98 (s, 2H, CH₂), 5.39-5.50 (q, *J*= 16 Hz, 2H, CH₂), 7.06-7.12 (m, 2H, Ar), 7.17-7.21 (m, 4H, Ar), 7.30-7.40 (m, 7H, Ar). **¹³C NMR (CDCl₃, 100 MHz) δ:** 35.85, 54.43, 75.96, 110.27, 122.57, 123.14, 123.77, 124.88, 125.65, 127.06, 128.24, 129.01, 129.31, 130.32, 130.57, 134.37, 140.76, 141.95, 143.00, 176.54. **MS (ESI) m/z:** 403.1 [M]⁺. **HPLC:** Daicel Chiralpak IA, n-hexane/i-propanol = 80/20, 1.0 mL/min, 210 nm, retention time: 26.960 min (minor), 34.227 min (major).

### 1-((1-Benzyl-1H-1,2,3-triazol-4-yl)methyl)-3-hydroxy-3-(3-hydroxyphenyl)indolin-2-one (S)-(6)

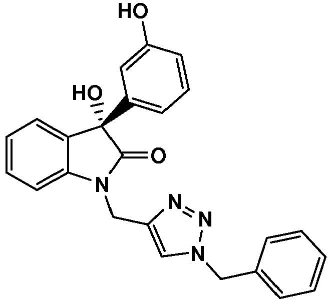

White solid. **m.p.=** 212.1-213.9 °C. **¹H NMR ((CD₃)₂SO, 400 MHz) δ:** 4.88-5.01 (q, *J*= 16 Hz, 2H, CH₂), 5.57 (s, 2H, CH₂), 6.56-6.58 (d, *J*= 8 Hz, 1H, Ar), 6.63-6.65 (d, *J*= 8 Hz, 1H, Ar), 6.68 (s, 1H, Ar), 6.80 (s, 1H, Ar), 7.01-7.05 (m, 2H, Ar), 7.11-7.16 (m, 2H, Ar), 7.26-7.37 (m, 6H, Ar), 8.10 (s, 1H, OH), 9.39 (s, 1H, OH). **¹³C NMR ((CD₃)₂SO, 100 MHz) δ:** 34.83, 52.80, 76.89, 109.46, 112.55, 114.47, 115.93, 122.78, 123.57, 124.42, 127.89, 128.15, 128.77, 129.07, 129.12, 133.17, 136.01, 142.13, 142.28, 142.69, 157.19, 176.30. **MS (ESI) m/z:** 413.1 [M]⁺. **HPLC:** Daicel Chiralpak IA, n-hexane/i-propanol = 80/20, 1.0 mL/min, 210 nm, retention time: 40.500 min (minor), 44.553 min (major).

### 1-((1-Benzyl-1H-1,2,3-triazol-4-yl)methyl)-5-bromo-3-hydroxy-3-phenylindolin-2-one (S)-(7)

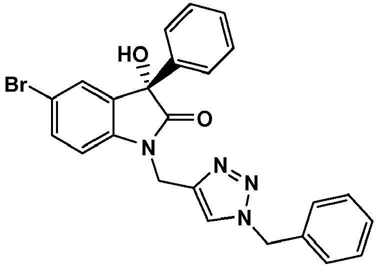

White solid. **m.p.=** 149.1-150.9 °C. **¹H NMR (CDCl₃, 400 MHz) δ:** 4.88-4.98 (q, *J*= 16 Hz, 2H, CH₂), 5.35-5.47 (q, *J*= 16 Hz, 2H, CH₂), 7.08-7.10 (d, *J*= 8 Hz, 1H, Ar), 7.20-7.21 (m, 2H, Ar), 7.29 (m, 4H, Ar), 7.34 (m, 4H, Ar), 7.41-7.42 (m, 2H, Ar, =CH), 7.50-7.54 (t, *J*= 8 Hz, 1H, Ar), 8.24-8.26 (d, *J*= 8 Hz, 1H, Ar) . **¹³C NMR (CDCl₃, 100 MHz) δ:** 35.76, 54.45, 77.92, 111.79, 116.59, 125.32, 128.12, 128.26, 128.71, 128.87, 129.03, 129.30, 132.90, 133.62, 134.28, 135.76, 139.37, 141.18, 142.50, 176.88. **MS (ESI) m/z:** 477.1 [M]⁺². **HPLC:** Daicel Chiralpak IA, n-hexane/i-propanol = 80/20, 1.0 mL/min, 210 nm, retention time: 24.347 min (minor), 47.020 min (major).

### 1-((1-Benzyl-1H-1,2,3-triazol-4-yl)methyl)-3-hydroxy-3-p-tolylindolin-2-one (S)-(8)

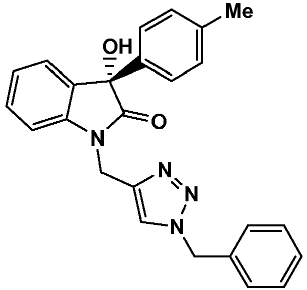

White solid. **m.p.=** 153.8-155.0 °C. **¹H NMR (CDCl₃, 400 MHz) δ:** 2.30 (s, 3H, CH₃), 4.96 (s, 2H, CH₂), 5.34-5.46 (q, *J*= 16 Hz, 2H, CH₂), 7.02-7.08 (m, 3H, Ar), 7.16-7.33 (m, 10H, Ar), 7.41 (s, 1H, =CH) . **¹³C NMR (CDCl₃, 100 MHz) δ:** 21.23, 35.79, 54.34, 77.89, 110.12, 122.65, 123.80, 124.97, 125.36, 128.19, 128.91, 129.24, 129.41, 130.03, 131.69, 134.43, 137.07, 138.25, 142.15,143.04, 177.47. **MS (ESI) m/z:** 411.2 [M]⁺. **HPLC:** Daicel Chiralpak IA, n-hexane/i-propanol = 80/20, 1.0 mL/min, 210 nm, retention time: 22.273 min (minor), 29.020 min (major).

### 1-((1-Butyl-1H-1,2,3-triazol-4-yl)methyl)-3-hydroxy-3-phenylindolin-2-one (S)-(9)

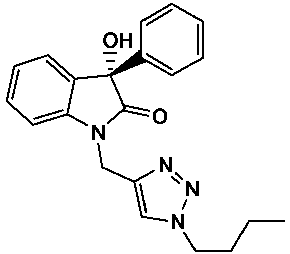

Pale yellow oil. **¹H NMR ((CD₃)₂CO, 400 MHz) δ:** 0.88-0.91 (t, 3H, CH₃), 1.24-1.32 (m, 2H, CH₂), 1.77-1.85 (m, 2H, CH₂), 4.32-4.36 (t, *J*= 8 Hz, 2H, CH₂), 4.92-5.08 (q, *J*= 16 Hz, 2H, CH₂), 5.88 (s br, 1H, OH), 7.01-7.05 (t, *J*= 8 Hz, 1H, Ar), 7.18-7.21 (m, 2H, Ar), 7.26-7.31 (m, 4H, Ar), 7.42-7.44 (d, *J*= 8 Hz, 2H, Ar), 7.87 (s, 1H, =CH) . **¹³C NMR ((CD₃)₂CO, 100 MHz) δ:** 13.68, 20.13, 32.86, 35.88, 50.27, 78.32, 110.42, 123.49, 123.62, 125.33, 126.38, 128.39, 128.88, 130.07, 133.79, 142.29, 143.13, 143.43, 177.28. **MS (ESI) m/z:** 363.2 [M]⁺. **HPLC:** Daicel Chiralpak IA, n-hexane/i-propanol = 80/20, 1.0 mL/min, 210 nm, retention time: 14.820 min (minor), 19.087 min (major).

### 1-((1-Benzyl-1H-1,2,3-triazol-4-yl)methyl)-3-(4-bromophenyl)-3-hydroxyindolin-2-one (10)

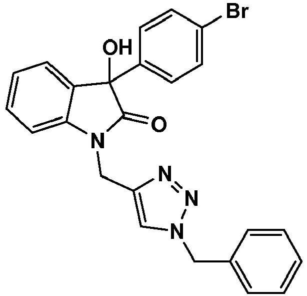

Pale yellow solid. **m.p.=** 152.1-153.0 °C. **¹H NMR (CDCl₃, 400 MHz) δ:** 4.97 (s, 2H, CH₂), 5.38-5.50 (q, *J*= 16 Hz, 2H, CH₂), 7.05-7.09 (t, *J*= 8 Hz, 1H, Ar), 7.16-7.22 (m, 6H, Ar), 7.31-7.41 (m, 7H, Ar). **¹³C NMR (CDCl₃, 100 MHz) δ:** 35.81, 54.46, 77.64, 110.32, 122.54, 122.65, 124.02, 124.96, 127.29, 128.27, 129.07, 129.33, 130.44, 131.09, 131.84, 134.33, 139.05, 142.18, 142.84, 176.79. **MS (ESI) m/z:** 477.1 [M]⁺². **HPLC:** Daicel Chiralpak IA, n-hexane/i-propanol = 80/20, 1.0 mL/min, 210 nm, retention time: 25.140 min (minor), 27.453 min (major).

### 1-((1-Benzyl-1H-1,2,3-triazol-4-yl)methyl)-3-hydroxy-5-methyl-3-phenylindolin-2-one (S)-(11)

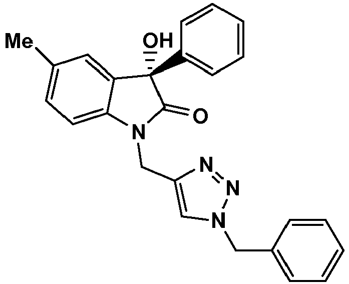

White solid. **m.p.=** 163.7-164.3 °C. **¹H NMR (CDCl₃, 400 MHz) δ:** 2.25 (s, 3H, CH₃), 3.44 (s br, 1H, OH), 4.96 (s, 2H, CH₂), 5.34-5.47 (q, *J*= 16 Hz, 2H, CH₂), 7.05-7.08 (m, 3H, Ar), 7.20 (m, 2H, Ar), 7.28-7.33 (m, 8H, Ar), 7.41 (s, 1H, =CH). **¹³C NMR (CDCl₃, 100 MHz) δ:** 21.16, 35.86, 54.36, 78.14, 109.98, 122.61, 125.35, 125.69, 128.20, 128.38, 128.72, 128.93, 129.25, 130.40, 131.56, 133.59, 134.42, 139.71, 140.19, 143.10, 177.32. **MS (ESI) m/z:** 411.2 [M]⁺. **HPLC:** Daicel Chiralpak IA, n-hexane/i-propanol = 80/20, 1.0 mL/min, 210 nm, retention time: 24.727 min (minor), 31.080 min (major).

### 1-((1-Benzyl-1H-1,2,3-triazol-4-yl)methyl)-3-(4-chlorophenyl)-3-hydroxyindolin-2-one (12)

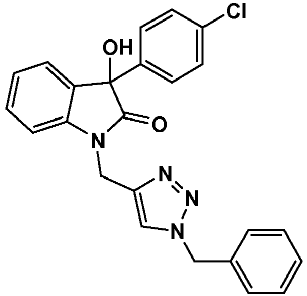

Pale yellow solid. **m.p.=** 141.4-142.8 °C. **¹H NMR (CDCl₃, 400 MHz) δ:** 4.98 (s, 2H, CH₂), 5.39-5.50 (q, *J*= 12 Hz, 2H, CH₂), 7.05-7.09 (t, *J*= 8 Hz, 1H, Ar), 7.21-7.23 (m, 8H, Ar), 7.31-7.35 (m, 4H, Ar), 7.41 (s, 1H, =CH). **¹³C NMR (CDCl₃, 100 MHz) δ:** 35.82, 54.46, 77.59, 110.32, 122.54, 124.01, 124.97, 126.99, 128.26, 128.90, 129.07, 129.33, 130.43, 131.14, 134.33, 134.45, 138.50, 142.19, 142.86, 176.87. **MS (ESI) m/z:** 431.1 [M]⁺. **HPLC:** Daicel Chiralpak IA, n-hexane/i-propanol = 80/20, 1.0 mL/min, 210 nm, retention time: 20.447 min (major), 23.687 min (minor).

### Biological Screening Studies 24 h MTT Cytototoxicity Assay - SW480 cell line

Approximately 5 × 10³ SW480 colon adenocarcinoma cells were cultured in 200 µl culture medium per well (DMEM medium), supplemented with 10% newborn calf serum and 1% amphotericin-penicillin-streptomycin solution in 96-well plates and incubated at 37 °C under a 5% CO₂ atmosphere. The cells were grown for 24h and then exposed to different concentrations of the tested drugs (dissolved in culture medium) for 24h. Hydrogen peroxide was used as a positive control. The cells were washed with the culture medium, incubated with MTT (500 µg/ml) for a further period of 3 hours. At the end of the incubation, the medium was removed, then carefully washed with PBS and 200 µl DMSO were added to each well. Absorbance was read at 570 nm in a microplate reader. Four replicates per dose were included. The IC50 values, that is, the concentrations which produced 50% inhibition of cell viability were calculated from MTT data using the GraphPadPrism Software Inc. (version 6.01) (USA).

### 72 h MTT assay on lymphoma cell lines.

Compounds were dissolved in dimethyl sulphoxide (DMSO) to obtain a stock concentration of 20 mM.

Cell lines. The panel of lymphoma cell lines comprised models derived from diffuse large B-cell lymphoma (n. 4) cell lines derived from DLBCL of the germinal center B-cell type (GCB-DLBCL), such as VAL and DOHH-2, and derived of the activated B-cell type (ABC-DLBCL) such as OCI-LY-10 and SU-DHL-2.

Proliferation MTT assay.

Cells were seeded in 96-well plates (non-tissue culture treated) at a density of 10,000 cells for all cell lines. Each compound was dissolved in dimethyl sulphoxide (DMSO). For treatment of cells, compounds were serially diluted in the appropriate tissue culture medium, at a range of 20 µM - 19 nM following a 1:2 dilution factor and added to cells (in three replicates). Cells were incubated for 72 hours at 37 °C, 5% CO₂. DMSO alone was added to negative control (untreated) cells. Wells containing medium only were included on each plate and served as blanks for absorbance readings. MTT (Sigma-Aldrich Chemie GmbH, Buchs, Switzerland) was prepared as a 5 mg/ml stock in PBS and filter-sterilized. MTT solution (22µL) was added to each well and tissue culture plates were incubated at 37°C for 4 hours. Cells were then lysed with 25% SDS lysis buffer and absorbance was read at 570 nm using an AD340 plate reader (Beckman Coulter International SA, Switzerland). Absorbance values were expressed in percentage of proliferating cells in comparison with control cells (control corresponds to 100%).

### Abbreviations

- DIPEA: Diisopropylethylamine
- DMA: Dimethylacetamide
- DMF: Dimethylforamide
- DMSO: Dimethylsulfoxide
- ee: Enantiomeric excess
- NHC: N-Heterocyclic Carbene ligands
- MTT: 3-(4,5-Dimethylthiazolyl-2)-2,5-diphenyltetrazoliumbromide
- NMP: N-Methylpiperidine
- THF: Tetrahydofuran

### References

M. A. Ali, R. Ismail, T. S. Choon, Y. K. Yoon, A. C. Wei, S. Pandian, R. S. Kumar, H. Osman, E. Manogaran, Bioorg. Med. Chem. Lett. 2010, 20, 7064.
Burke, A.J.; Marques, C.S. Chiral Non-racemic *N*-(1,2,3-triazolmethyl)-3-hydroxy-3-aryloxindoles, Prov. Patent Application, 109598, 26 Aug 2016.
C.E. DeSantis, C. C. Lin, A.B. Mariotto, R.L. Siegel, K. D. Stein, J. L., Kramer, R. Alteri, A.S. Robbins, A. Jemal, Cancer J. Clin. 2014, 64, 252.
C. S.-L. Gal, J. Wagnon, J. Simiand, G. Griebel, C. Lacour, G. Guillon, C. Barberis, G. Brossard, P. Soubrié , D. Nisato, M. Pascal, R. Pruss, B. Scatton, J.-P. Maffrand, G. Le Fur, J. Pharmacol. Exp. Ther., 2002, 300, 1122.
L. Kelland, Nat. Rev. Cancer 2007, 7, 8, 573.
H.C. Neu, K.P. Fu, Antimicrob. Agents and Chemotherapy, 1979, 209.
R. Ng, Drugs - From Discovery to Approval, John Wiley & Sons, Inc, Hoboken, New Jersey, 2004**.**
   J. McCauley, A. Zivanovic, D. Skropeta, Methods Mol. Biol. 2013, 1055, 191.
M. Ochi, K. Kawasaki, H. Kataoka, Y. Uchio, H. Nishi, Biochem. Biophys. Res. Commun. 2001, 283, 1118.
F.G.E. Perabo, A. Wirger, S. Kamp, H. Lindner, D.H. Schmidt, S.C. Müller, E.C. Kohn, Anticancer Res. 2004, 24, 2869.
S. Rossi, (ed.), Australian Medicines Handbook, Adelaide: The Australian Medicines Handbook Unit Trust, 2013**.**
V.V. Rostovtsev, L.G. Green, V.V. Fokin, K.B. Sharpless, Angew. Chem. Int. Ed. 2002, 41, 2596.
M. Rottmann, C. McNamara, B. K. S. Yeung, M. C. S. Lee, B. Zou, B. Russell, P. Seitz, D. M. Plouffe, N. V. Dharia, J. Tan, S. B. Cohen, K. R. Spencer, G. E. González-Páez, S. B. Lakshminarayana, A. Goh, R. Suwanarusk, T. Jegla, E. K. Schmitt, H.-P. Beck, R. Brun, F. Nosten, L. Renia, V.
Dartois, T. H. Keller, D. A. Fidock, E. A. Winzeler, T. T. Diagana, Science, 2010, 329, 1175.
P. Thirumurugan, D. Matosiuk, K. Jozwiak, Chem. Rev. 2013, 113, 4905.
C.W. Tornøe, C. Christensen, M. Meldal, J. Org. Chem. 2002, 67, 3057.
J. Totobenazara, A.J. Burke, Tetrahedron Letters, 2015, 56, 2853.
J. Totobenazara, A.A. San Juan, P. Bacalhau, C.S. Marques, A. Goth, M. R. Martins, A. T. Caldeira, A.J. Burke, ChemistrySelect, 2016, 1, 3580.
Y. Yang, B.A., Rasmussen, D.M. Shlaes, Pharmacol Ther., 1999, 83, 141*.*
G.C. Tron, T.Pirali, R.A. Billington, P.L. Canonico, G. Sorba, A.A. Genazzani, Med. Res. Rev. 2008, 28, 278.
C. Widakowich, G. de Castro, E. de Azambuja, P. Dinh, A. Awada, Oncologist 2007, 12, 1443.
L.-H. Xu, C.-S. Deng, Y.-Q. Zhu, S-Q. Liu, D.-Z. Liu, World J. Gastroenterol, 2003, 9, 1241.

## Claims

1. Compounds of formula (**I**) and (**II**) for use in the inhibition of colon adenocarcinoma and lymphoma cells, wherein 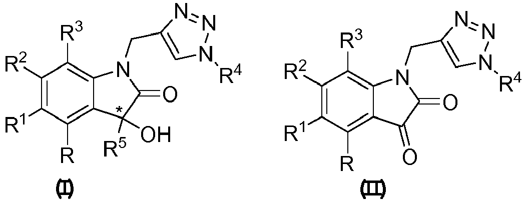
R, R¹, R² and R³ represent; H, alkyl, aryl, vinyl, allyl, alkoxyl, halogen (F, Br, Cl), OH, CN, CHO and CO₂H,
R⁴ represents a linear alkyl group with 1-6 carbons, a cycloheteroalkyl group, like: 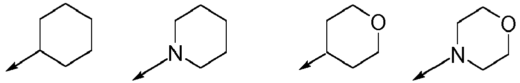 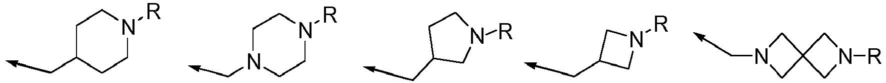 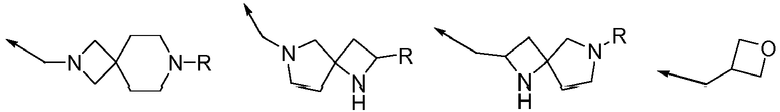 an aryl group or heteroaryl group, that includes, a substituted phenyl group (containing alkoxyl, halogen (F, Br, Cl), OH, CN, CHO, CO₂H), 2- and 4-pyridine, pyrimidine, pyridazine, thiophene, furane, pyran, benzoyl, pyrrole;
A benzyl group 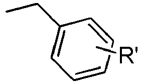
where R' is an alkoxyl, halogen (F, Br, Cl), OH, CN, CHO and CO₂H,
R⁵ represents an aryl group.

2. Compounds for the use in the inhibition of colon adenocarcinoma and lymphoma cells according to the previous claim, wherein the compounds are used as the appropriate individual enantiomers.

3. Compounds for the use in the inhibition of colon adenocarcinoma and lymphoma cells according to claim 1, wherein the compounds are used as a racemic mixture.

4. Compounds for use in the inhibition of colon adenocarcinoma and lymphoma cells according to claim 1, wherein the lymphoma cell lines include B-cell lymphoma (n. 4) derived from DLBCL of the germinal center B-cell type (GCB-DLBCL), such as VAL and DOHH-2, and derived from the activated B-cell type (ABC-DLBCL) such as OCI-LY-10 and SU-DHL-2.

## Patentansprüche

1. Verbindungen von Formel (I) und (II) zur Verwendung bei der Hemmung von Adenokarzinom-Darmzellen und Lymphomzellen, worin 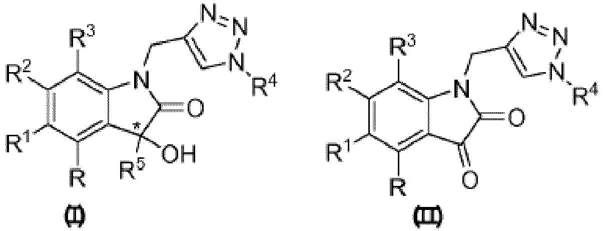
R, R¹, R² und R³ Wasserstoff, Alkyl, Aryl;, Vinyl, Allyl, Alkoxyl, Halogen darstellen (F, Br, Cl); OH, CN, CHO und CO₂H,
R⁴ stellen eine lineare Alkylgruppe mit 1-6 Kohlenstoffen, eine Zykloheteroalkyl-Gruppe dar, wie: 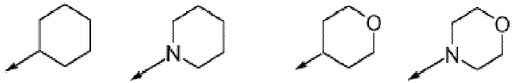 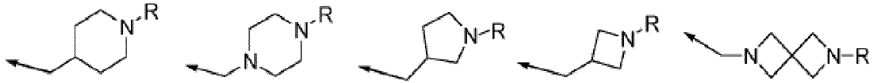 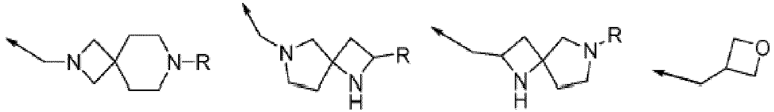 eine Arylgruppe oder Heteroarylgruppe, welche eine substituierte Phenylgruppe beinhaltet (bestehend aus Alkoxyl, Halogen (F, Br, Cl), OH, CN, CHO, CO₂H), 2- und 4-Pyridin, Pyrimidin, Pyridazin, Thiophen, Furan, Pyran, Benzoyl, Pyrrol;
Eine Benzylgruppe 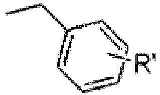
wo R' ein Alkoxyl, Halogen (F, Br, Cl), OH, CN, CHO und CO₂H ist,
R⁵ eine Arylgruppe darstellt.

2. Verbindungen zur Verwendung bei der Hemmung von Adenokarzinom-Darmzellen und Lymphomzellen gemäß dem vorherigen Anspruch, worin die Verbindungen als die entsprechenden individuellen Enantiomere verwendet werden.

3. Verbindungen zur Verwendung bei der Hemmung von Adenokarzinom-Darmzellen und Lymphomzellen gemäß Anspruch 1, worin die Verbindungen als ein racemisches Gemisch verwendet werden.

4. Verbindungen zur Verwendung bei der Hemmung von Adenokarzinom-Darmzellen und Lymphomzellen gemäß Anspruch 1, worin die Lymphom Zellinien B-Zellen-Lymphom (No. 4) beinhalten, entzogen aus DLBCL des Keimzentrums vom B-Zelltyp (GCB-DLBCL), wie VAL und DOHH-2 und entzogen aus dem aktivierten B-Zelltyp (ABC-DLBCL), wie OCI-LY-IO und SU-DHL-2.

## Revendications

1. Composés de formule (I) et (II) pour utilisation dans l'inhibition de cellules d'adénocarcinome et de lymphome du côlon, dans lesquels 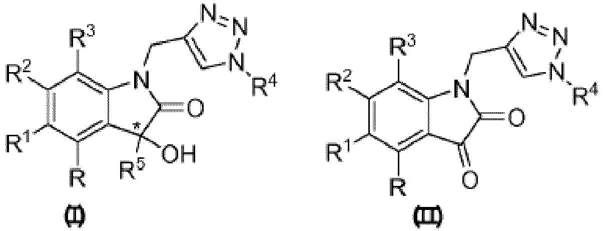
R, R¹, R² et R³ représentent ; H, alkyle, aryle, vinyle, allyle, alcoxyle, halogène (F, Br, Cl), OH, CN, CHO et CO₂H,
R⁴ représentent un groupe alkyle linéaire avec 1-6 carbones, un groupe cyclohétéroalkyle, tel que : 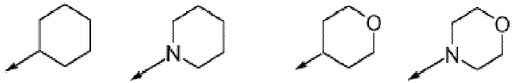 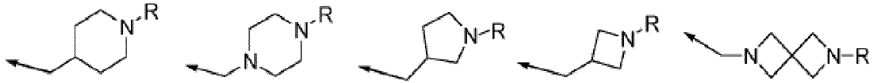 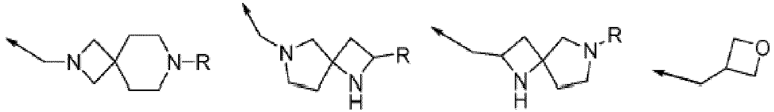 un groupe aryle ou un groupe hétéroaryle, qui comprend un groupe phényle substitué (contenant de l'alcoxyle, de l'halogène (F, Br, Cl), OH, CN, CHO, CO₂H), 2- et 4-pyridine, pyrimidine, pyridazine, thiophène, furane, pyrane, benzoyle, pyrrole ;
Un groupe benzyle 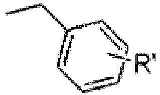
où R' est un alcoxyle, un halogène (F, Br, Cl), OH, CN, CHO et CO₂H,
R⁵ représente un groupe aryle.

2. Composés pour l'utilisation dans l'inhibition de cellules d'adénocarcinome et de lymphome du côlon selon la revendication précédente, dans lesquels les composés sont utilisés en tant qu'énantiomères individuels appropriés.

3. Composés pour l'utilisation dans l'inhibition de cellules d'adénocarcinome et de lymphome du côlon selon la revendication 1, dans lesquels les composés sont utilisés comme un mélange racémique.

4. Composés destinés à être utilisés dans l'inhibition des cellules d'adénocarcinome et de lymphome du côlon selon la revendication 1, dans lesquels les lignées cellulaires de lymphome comprennent des cellules de lymphome B (n. 4) dérivées de LDGCB du type cellule B à centre germinal (GCB-LDGCB)., tel que VAL et DOHH-2, et dérivées du type de cellule B activée (ABC-LDGBC) tel que OCI-LY-IO et SU-DHL-2.
